# EUROPEAN PATENT SPECIFICATION

(11) **EP 4 335 367 B1**
(45) Date of publication and mention of the grant of the patent: **24.09.2025**
(21) Application number: 23192905.0
(22) Date of filing: 23.08.2023
(51) Int. Cl.: A61B 5/145

(54) **RECYCLABLE DEVICE FOR DEPLOYING TRANSCUTANEOUS SENSORS AND RELATED TECHNOLOGY**
WIEDERVERWENDBARE VORRICHTUNG ZUM EINSATZ TRANSKUTANER SENSOREN UND ZUGEHÖRIGE TECHNOLOGIE
DISPOSITIF RECYCLABLE POUR DÉPLOYER DES CAPTEURS TRANSCUTANÉS ET TECHNOLOGIE ASSOCIÉE

(30) Priority: 07.09.2022 US 202217930176
(43) Date of publication of application: 13.03.2024
(73) Proprietor: Medtronic MiniMed, Inc., Northridge, CA 91325 (US)
(72) Inventor: GARAI, Ellis, Northridge, 91325 (US)
(74) Representative: Zimmermann & Partner Patentanwälte mbB

(56) References cited:
- US-A1- 2020 359 964
- US-A1- 2022 151 524
- US-B2- 11 219 716

## Description

### TECHNICAL FIELD

This disclosure is related to deployment of transcutaneous devices, such as transcutaneous blood-glucose sensors.

### BACKGROUND

Diabetes is a disease in which the body does not produce or properly use insulin. Millions of people in the United States and around the world have been diagnosed with some form of diabetes. Type 1 diabetes results from the body's failure to produce insulin. Type 2 diabetes results from insulin resistance in which the body fails to properly use insulin. In order to effectively manage the disease, diabetics must closely monitor and manage their blood glucose levels through exercise, diet, and medications. In particular, both Type 1 and some Type 2 diabetics rely on insulin delivery and blood glucose monitoring to control their diabetes.

Monitoring blood glucose levels plays an integral role in the management and control of diabetes. Finger stick measurements, glucose sensors and monitors have traditionally been used to check the blood glucose levels of diabetic patients. In recent years, continuous glucose monitoring systems have been developed utilizing the latest sensor technologies incorporating both implantable and external sensors. Newer systems deliver the preciseness of finger stick measurements coupled with the convenience of not having to repeatedly prick the skin to obtain glucose measurements. These newer systems provide the equivalent of over 200 finger stick readings per day. Additionally, continuous glucose monitoring systems enable physicians and patients to monitor blood glucose trends of their body and suggest and deliver insulin based on each patient's particular needs. Accordingly, physicians and medical device companies are always searching for more convenient ways to keep diabetic patients aware of their blood glucose levels throughout the day.

As such, analyte sensors may be generally used to test analyte levels in patients. For example, thin film sensors may be used for obtaining an indication of blood glucose levels and monitoring blood glucose levels in a diabetic patient. In these instances, a portion of a glucose sensor is positioned subcutaneously in direct contact with patient extracellular fluid. To insert a glucose sensor subcutaneously, an insertion device is used that quickly injects the sensor into the patient's skin and simultaneously adheres the monitor to the patient's skin. Glucose sensors need to be changed every few days, and whenever the sensor stops working, is damaged, or is giving erroneous readings. Deploying a new sensor involves puncturing the skin and securing the device at the side of the puncture. A tool called a "serter" is often used to facilitate this process. One issue with current serters is that they are typically single use devices that are disposed of every time a user inserts a new glucose sensor, which is every typically every 7 to 16 days. Further, current serters are mixed material devices, and thus are not suitable for easy recycling. Given that diabetes affects hundreds of millions of people worldwide and is only one example of a disease that benefits from convenient deployment of transcutaneous devices, there is an ongoing need to improve serters. Even small improvements in this field can have major public health benefits.
US 2022/0151524 A1 relates to transcutaneous analyte sensor systems and methods. US 11,219,716 B2 relates to a supplementary device for an injection device. US 2020/0359964 A1 relates to an analyte sensor.

### SUMMARY

In one aspect, the present disclosure describes a deployment device including a housing having a plunger, a frame movably connected to the plunger and defining a deployment window and a cap releasably connected to the plunger. The deployment device also includes a spring confined within the housing by the cap, wherein the plunger and the frame are resiliently connected to one another via the spring, and wherein the spring urges the frame to move in a first direction relative to the plunger. The deployment device also includes a carrier movably connected to the frame, wherein the carrier is configured to move a transcutaneous device into contact with a target surface of a subject via the deployment window at least partially in response to the frame moving in a second direction relative to the plunger, the second direction being opposite the first direction. The deployment device also includes a stop, that, when engaged, is configured to block separation of the cap from the plunger, wherein the stop is configured to disengage at least partially in response to the frame moving in the second direction relative to the plunger.

In further aspects, the frame carries the stop. In further aspects, one of the plunger and the cap includes an annular sealing surface, and the other of the plunger and the cap includes an annular flange configured to resiliently engage the sealing surface when the cap is connected to the plunger. In further aspects, the spring is removable from the housing when the cap is separated from the plunger. In further aspects, the cap and the plunger include complementary threads through which the cap and the plunger are releasably connected to one another. In further aspects, the stop, when engaged, is configured to block rotation of the cap relative to the plunger. In further aspects, the cap includes a support and a projection extending outwardly from the support in a plane perpendicular to the first and second directions. In further aspects, the stop, when engaged, is circumferentially adjacent to the projection along a circle within the plane.

In another aspect, the present disclosure describes a deployment device including a housing having a plunger and a frame movably connected to the plunger and defining a deployment window. The deployment device also includes a spring confined within the housing by the frame, wherein the plunger and the frame are resiliently connected to one another via the spring, and wherein the spring urges the frame to move in a first direction relative to the plunger. The deployment device also includes a carrier movably connected to the frame, wherein the carrier is configured to move a transcutaneous device into contact with a target surface of a subject via the deployment window at least partially in response to the frame moving in a second direction relative to the plunger, the second direction being opposite the first direction. The deployment device also includes a stop that, when engaged, is configured block separation of the frame from the plunger, wherein the stop is configured to disengage at least partially in response to the frame moving in the second direction relative to the plunger and then rotating in a third direction relative to the plunger, the third direction being perpendicular to the first and second directions.

In further aspects, the frame is configured to separate from the plunger at least partially in response to the frame moving in the first direction relative to the plunger after rotating in the third direction relative to the plunger. In further aspects, the frame defines a channel extending along a path parallel to the first and second directions. In further aspects, the plunger includes a key extending into the channel in a plane perpendicular to the path. In further aspects, the carrier is configured to move the transcutaneous device into contact with the target surface via the deployment window at least partially in response to the frame moving in the second direction relative to the plunger. In further aspects, the frame defines a range of motion in the second direction relative to the plunger. In further aspects, moving the frame in the second direction relative to the plunger through the full range of motion at least partially causes the key to move within the channel between a first end of the path and an opposite second end of the path. In further aspects, the channel and the path are a first channel and a first path, respectively, and the frame defines a second channel extending along a second path that branches from the first path at a branch point between the first and second ends of the first path. In further aspects, the stop is configured to disengage at least partially in response to the frame moving in the second direction relative to the plunger while the key moves along a portion of the first path beginning at the first end of the first path, and the frame then moving in the third direction relative to the plunger while the key moves along a portion of the second path beginning at the branch point. In further aspects, the first channel is closed ended at the first end of the first path, and the second channel is a through channel. In further aspects, the second path includes a step. In further aspects, the second channel includes a mouth at the branch point, the mouth being spaced apart from the first end of the first path by at least 10% of a length of the first path, and the mouth being spaced apart from the second end of the first path by at least 10% of the length of the first path. In further aspects, the frame includes a first nub at a first side of the mouth in a dimension parallel to the first path, and a second nub at an opposite second side of the mouth in the dimension parallel to the first path. In further aspects, the first and second nubs are configured to guide the key away from the mouth while the key moves from the first end of the first path to the second end of the first path, while the key moves from the second end of the first path to the first end of the first path, or both.

In another aspect, the present disclosure describes a method for connecting a transcutaneous device to skin of a subject. The method includes contacting a target surface of the subject and a frame of a housing of a deployment device while the transcutaneous device is staged within the housing and while the housing is locked. The method also includes moving a plunger of the housing toward the target surface in a first direction relative to the frame while the frame is in contact with the target surface and while the frame moves in a second direction relative to the plunger, the second direction being opposite the first direction, wherein moving the plunger toward the target surface in the first direction relative to the frame while the frame is in contact with the target surface at least partially causes the transcutaneous device to move into contact with the target surface. The method also includes moving the plunger away from the target surface after the transcutaneous device moves into contact with the target surface and while a spring of the deployment device moves the frame in the first direction relative to the plunger. The method also includes moving the frame in the second direction relative to the plunger after moving the plunger away from the target surface, wherein moving the frame in the second direction relative to the plunger after moving the plunger away from the target surface at least partially causes the housing to unlock. The method also includes opening the housing after the housing unlocks and removing the spring from the housing after opening the housing.

In further aspects, opening the housing includes rotating a cap of the housing relative to the plunger. In further aspects, contacting the target surface and the frame includes contacting the target surface and the frame while a stop of the deployment device blocks rotation of the cap relative to the plunger. In further aspects, moving the frame in the second direction relative to the plunger after moving the plunger away from the target surface at least partially causes the housing to unlock by disengaging the stop. In further aspects, removing the spring includes tilting the housing to allow the spring to drop from the housing by gravity. In further aspects, contacting the target surface and the frame includes contacting the target surface and the frame while a needle of the deployment device is staged within the housing. In further aspects, moving the plunger toward the target surface in the first direction relative to the frame while the frame is in contact with the target surface at least partially causes the needle to move toward and to pierce the target surface and the skin. In further aspects, moving the plunger toward the target surface in the first direction relative to the frame while the frame is in contact with the target surface at least partially causes the needle to withdraw from the skin, to move away from the target surface, and to move into a cartridge within the housing. In further aspects, the method further comprises removing the needle and the cartridge from the housing after opening the housing.

In another aspect, the present disclosure describes a method for connecting a transcutaneous device to skin of a subject. The method includes contacting a target surface of the subject and a frame of a housing of a deployment device while the transcutaneous device is staged within the housing. The method also includes moving a plunger of the deployment device toward the target surface in a first direction relative to the frame while the frame is in contact with the target surface and while the frame moves in a second direction relative to the plunger, the second direction being opposite the first direction, wherein moving the plunger toward the target surface in the first direction relative to the frame while the frame is in contact with the target surface at least partially causes the transcutaneous device to move into contact with the target surface. The method also includes moving the plunger away from the target surface after the transcutaneous device moves into contact with the target surface and while a spring of the deployment device moves the frame in the first direction relative to the plunger. The method also includes moving the frame in the second direction relative to the plunger after moving the plunger away from the target surface. The method also includes rotating the frame in a third direction relative to the plunger after moving the frame in the second direction relative to the plunger, the third direction being perpendicular to the first and second directions. The method also includes separating the frame from the plunger after rotating the frame in the third direction relative to the plunger. The method also includes removing the spring from the housing after separating the frame from the plunger.

In further aspects, the method also includes moving the frame in the first direction relative to the plunger while separating the frame from the plunger. In further aspects, removing the spring includes allowing the spring to drop from the housing by gravity. In further aspects, moving the plunger toward the target surface in a first direction relative to the frame while the frame is in contact with the target surface includes moving the plunger toward the target surface in a first direction relative to the frame while the frame is in contact with the target surface and while a key of the plunger moves along a path within a channel defined by the frame. In further aspects, the channel and the path are a first channel and a first path, respectively, moving the plunger away from the target surface after the transcutaneous device moves into contact with the target surface includes moving the plunger away from the target surface after the transcutaneous device moves into contact with the target surface and while the key moves along the first path within the first channel. In further aspects, rotating the frame in the third direction relative to the plunger after moving the frame in the second direction relative to the plunger includes rotating the frame in the third direction relative to the plunger after moving the frame in the second direction relative to the plunger and while the key moves along a second path within a second channel defined by the frame. In further aspects, the second path branches from the first path. In further aspects, the method also includes blindly aligning the key with a mouth of the second channel while moving the frame in the second direction relative to the plunger after moving the plunger away from the target surface.

Further disclosed herein is a deployment device for deploying a transcutaneous sensor in accordance with an embodiment of the present technology which includes a housing including a plunger, a frame, and a cap. The deployment device further includes a spring and a carrier within the housing. The frame defines a deployment window through which the carrier is configured to move the transcutaneous device into contact with a target surface of a subject. This can occur at least partially in response to the frame moving in a second direction relative to the plunger, the second direction being opposite the first direction. The deployment device also include a stop that, when engaged, is configured block separation of the cap from the plunger, thereby enabling the spring to be removed from the housing. The stop is configured to disengage at least partially in response to the frame moving in the second direction relative to the plunger.

### BRIEF DESCRIPTION OF THE DRAWINGS

Many aspects of the present technology can be better understood with reference to the following drawings. The relative dimensions in the drawings may be to scale with respect to some embodiments of the present technology. With respect to other embodiments, the drawings may not be to scale. The drawings may also be enlarged arbitrarily. For clarity, reference-number labels for analogous components or features may be omitted when the appropriate reference-number labels for such analogous components or features are clear in the context of the specification and all of the drawings considered together. Furthermore, the same reference numbers may be used to identify analogous components or features in multiple described embodiments.
FIG. 1 is an exploded perspective view of a system including a deployment device and a protective cover in accordance with at least some embodiments of the present technology.
FIG. 2 is a perspective view of the deployment device shown in FIG. 1.
FIGS. 3A-3D are profile views of different respective times during use of the deployment device shown in FIG. 1 to deploy a sensor.
FIG. 4 is an exploded profile view of the deployment device shown in FIG. 1.
FIG. 5 is a perspective view of a cap of the deployment device shown in FIG. 1.
FIG. 6 is a perspective view of a frame of the deployment device shown in FIG. 1.
FIG. 7 is a top plan view of the deployment device shown in FIG. 1.
FIG. 8 is a cross-sectional perspective view of a portion of the deployment device shown in FIG. 1 taken along the line A-A in FIG. 7.
FIG. 9 is an exploded perspective view of another system including a deployment device and a protective cover in accordance with at least some embodiments of the present technology.
FIG. 10 is a perspective view of the deployment device shown in FIG. 9.
FIG. 11 is an exploded profile view of the deployment device shown in FIG. 9.
FIG. 12 is a top plan view of a plunger of the deployment device shown in FIG. 9.
FIG. 13 is a cross-sectional perspective view of the plunger of the deployment device shown in FIG. 9 taken along the line B-B in FIG. 12.
FIG. 14 is a perspective view of a frame of a housing of the deployment device shown in FIG. 9.
FIG. 15 is a schematic illustration of first and second channels defined by the frame of the deployment device shown in FIG. 9.
FIG. 16 is a perspective view of a frame of a housing of another deployment device in accordance with at least some embodiments of the present technology.
FIG. 17 is a schematic illustration of first and second channels defined by the frame shown in FIG. 16.
FIG. 18 is a perspective view of a frame of a housing of yet another deployment device in accordance with at least some embodiments of the present technology.
FIG. 19 is a schematic illustration of first and second channels defined by the frame shown in FIG. 18.
FIG. 20 is a block diagram illustrating a method for connecting a transcutaneous device to skin of a subj ect in accordance with at least some embodiments of the present technology.

### DETAILED DESCRIPTION

Disclosed herein are recyclable devices for deploying transcutaneous sensors and related devices, systems, and methods. Devices in accordance with at least some embodiments of the present technology include innovative features that enable device components made of dissimilar materials to be separated from one another after use. Deployment devices are typically single-use and disposable for reasons of safety and convenience. Frequent disposal of used deployment devices, however, is wasteful and environmentally harmful. At the same time, conventional deployment devices are not recyclable by standard recycling systems because such devices include integrated components made of dissimilar materials. For example, metal is typically the most practical material for springs that transfer force within a deployment device to enable the device to rapidly insert and retract a needle. In contrast, plastic is typically the most practical material for a housing of a deployment device. Most recycling systems require that consumers separate metal waste from plastic waste because the processes for recycling these materials are different. Accordingly, products that include metal and plastic components that are difficult to separate must be disposed of as garbage rather than recycled.

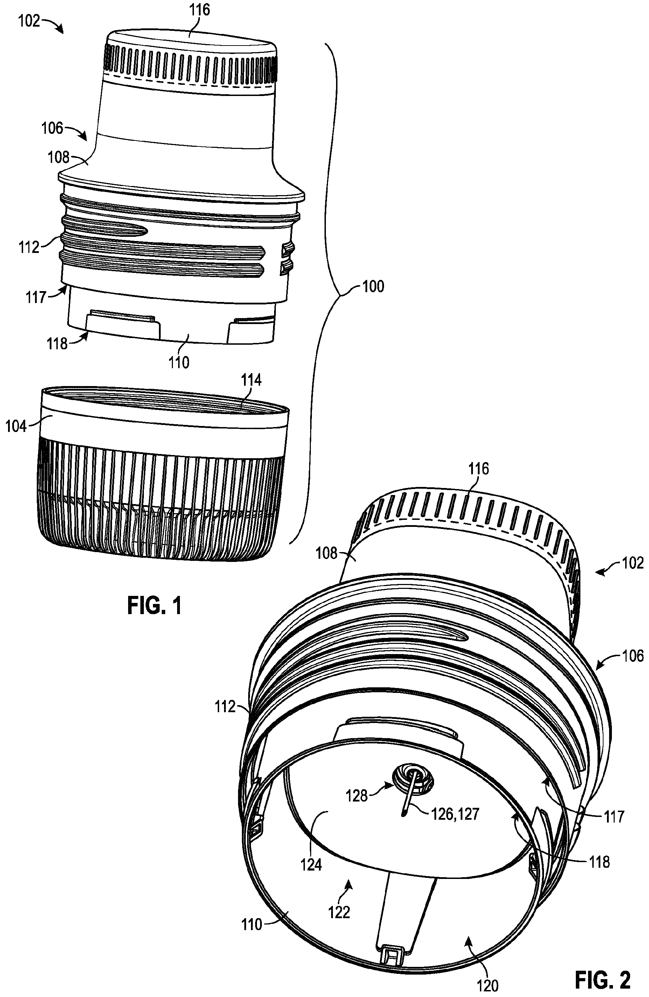

Designing a deployment device that enables components made of dissimilar materials to be conveniently separated from one another after use to facilitate recycling is a challenging problem. Complicating factors include the presence of many moving parts and the need to prevent parts from being separated accidentally or prematurely. For example, opening a deployment device prematurely may cause the deployment device to malfunction, may cause the deployment device to be difficult to reassemble, may cause a component (e.g., a spring) within the deployment device to eject forcefully, and/or may have one or more other undesirable effects. Devices in accordance with at least some embodiments of the present technology address these and/or other challenges by including housings that default to a locked state before use. In addition or alternatively, devices in accordance with at least some embodiments of the present technology include housings that open via two or more operations that an end user is unlikely to perform unintentionally. These and other features of deployment devices and associated systems and methods in accordance with various embodiments of the present technology are further described below with reference to FIGS. 1-20.

Although systems, devices, and methods may be described herein primarily or entirely in the context of deploying transcutaneous blood-glucose sensors, other contexts are within the scope of the present technology. For example, suitable features of described systems, devices, and methods for deploying transcutaneous blood-glucose sensors can be implemented in the context of deploying infusion patch pumps or deploying other transcutaneous devices. Furthermore, it should be understood, in general, that other systems, devices, and methods in addition to those disclosed herein are within the scope of the present disclosure. For example, systems, devices, and methods in accordance with embodiments of the present technology can have different and/or additional configurations, components, procedures, etc. than those disclosed herein. Moreover, systems, devices, and methods in accordance with embodiments of the present disclosure can be without one or more of the configurations, components, procedures, etc. disclosed herein without deviating from the present technology.

FIG. 1 is an exploded perspective view of a system 100 including a deployment device 102 and a protective cover 104 in accordance with at least some embodiments of the present technology. FIG. 2 is a perspective view of just the deployment device 102. With reference to FIGS. 1 and 2 together, the deployment device 102 can include a housing 106 including a plunger 108 and a frame 110 nested within and movably connected to the plunger 108. When a consumer receives the system 100, threads 112 on an outer surface of the plunger 108 and complementary threads 114 on an inner surface of the protective cover 104 can be engaged to releasably connect the housing 106 and the protective cover 104 to one another. The housing 106 can further include a cap 116 connected to the plunger 108. As with the protective cover 104 and the plunger 108, the cap 116 and the plunger 108 can be connected to one another via complementary threads (not shown). The deployment device 102 can be configured to maintain a sterile environment within the housing 106 while the protective cover 104 and the cap 116 are in place. In its default state before use, the housing 106 can be locked. For example, rotation of the cap 116 relative to the plunger 108 is blocked. Also in the default state, the frame 110 can project beyond an annular lowermost edge 117 of the plunger 108.

As shown in FIG. 2, the frame 110 can have an annular lowermost edge 118 at which the frame 110 defines a deployment window 120. Before use, the deployment device 102 can include a sensor 122 or other suitable transcutaneous device staged within the housing 106. The sensor 122 can include a patch 124 configured to adhesively or otherwise connect the sensor 122 to a target surface. The target surface, for example, can be the surface of a subject's skin or the surface of another material or structure attached to a subject's skin. In some cases, an end user may need to remove a release liner to expose adhesive on the patch 124 before use. In other cases, removing the protective cover 104 exposes adhesive on the patch 124 automatically. When staged within the housing 106, the sensor 122 can be positioned such that the patch 124 is spaced apart upwardly from the deployment window 120. This spacing can be greater than a spacing between the annular lowermost edge 118 of the frame 110 and the annular lowermost edge 117 of the plunger 108 when the deployment device 102 is in its default state. The deployment device 102 can further include a needle 126 that extends through an opening 128 of the sensor 122 and projects downwardly beyond the patch 124. In at least some cases, the sensor 122 includes a cannula 127 coaxially disposed around the needle 126.

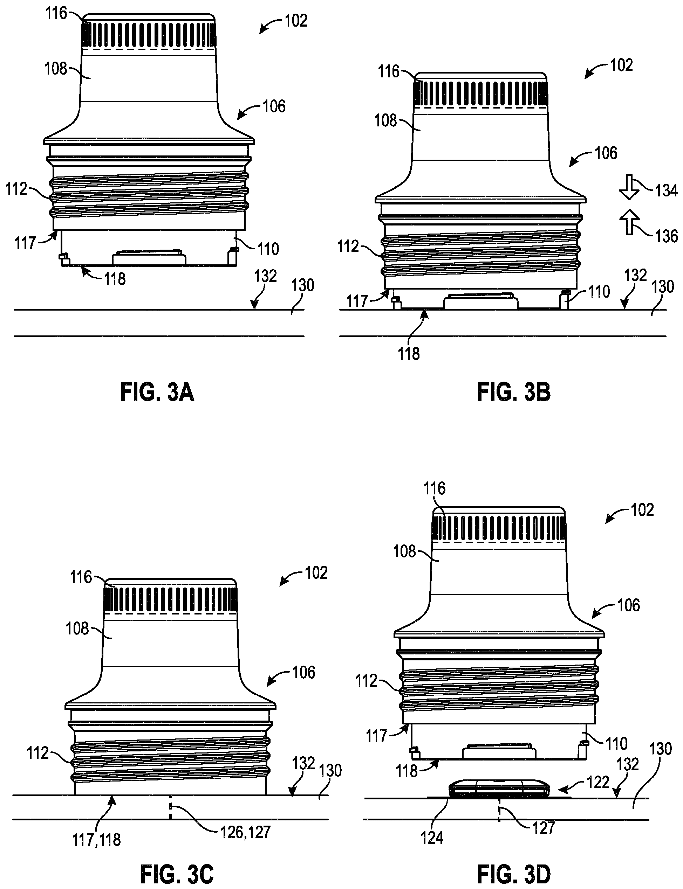

FIGS. 3A-3D are profile views of different respective times during use of the deployment device 102 to deploy the sensor 122. With reference to FIGS. 3A-3D together, the deployment device 102 can first be positioned near a region of skin 130 defining a target surface 132. In the illustrated case, the target surface 132 is a bare surface of the skin 130. In other cases, a counterpart of the target surface 132 is a surface of a material or structure attached to the skin 130. As shown in FIG. 3B, the deployment device 102 can then be moved toward the target surface 132 such that the annular lowermost edge 118 of the frame 110 moves into contact with the target surface 132. Force from the target surface 132 can then cause the plunger 108 and the frame 110 to move relative to one another. In particular, the plunger 108 can move in a first direction 134 relative to the frame 110 while the frame 110 moves in an opposite second direction 136 relative to the plunger 108. During at least some of this relative movement, the sensor 122 can remain with the plunger 108. Thus, the frame 110 can move relative to both the plunger 108 and the sensor 122.

As shown in FIG. 3C, relative movement between the plunger 108 and the frame 110 can cause a spring mechanism within the housing 106 to rapidly and forcefully move the sensor 122, the needle 126, and the cannula 127 toward the target surface 132 relative to both the plunger 108 and the frame 110. This action can cause the needle 126 and the cannula 127 to be inserted into the skin 130 and the patch 124 to adhesively connect to the target surface 132. Another spring mechanism within the housing 106 can then cause the needle 126 to rapidly withdraw from the skin 130 and to move in the second direction 136 relative to the plunger 108 and the frame 110. As shown in FIG. 3D, the deployment device 102 can then be moved away from the skin 130. During this movement, the plunger 108 can move in the second direction 136 relative to the frame 110 while the frame 110 moves in the first direction 134 relative to the plunger 108 until the frame 110 is no longer in contact with the target surface 132. Due to the adhesive connection between the patch 124 and the target surface 132, the sensor 122, including the cannula 127, can remain connected to the skin 130 after the deployment device 102 is removed. At least some of the these and/or other aspects of operation of the deployment device 102 are discussed in U.S. Patent No. 10,413,183, which in incorporated herein by reference in its entirety. To the extent this reference conflicts with the present disclosure, the present disclosure controls.

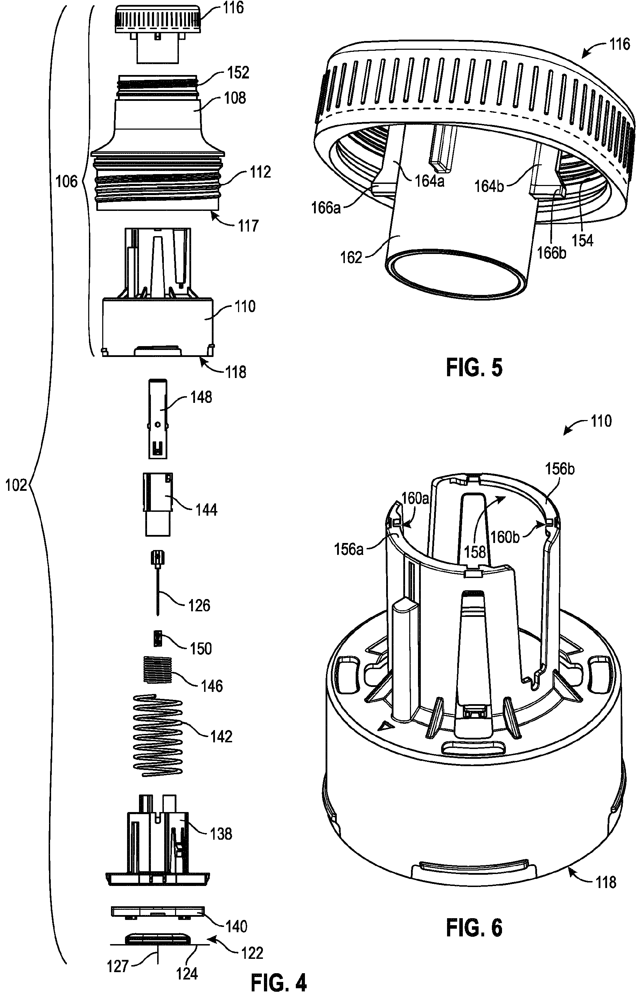

FIG. 4 is an exploded profile view of the deployment device 102. With reference to FIGS. 3A-4 together, the deployment device 102 can include a carrier 138 nested within and movably connected to the frame 110. The deployment device 102 can further include a retainer 140 configured to releasably connect the sensor 122 to the carrier 138. The carrier 138 can be configured to move the sensor 122 into contact with the target surface 132 via the deployment window 120 at least partially in response to the frame 110 moving in the second direction 136 relative to the plunger 108. The deployment device 102 can include a first spring 142 that powers this movement of the carrier 138. The first spring 142 can be configured to store enough force to drive the needle 126 and the cannula 127 into the skin 130. The first spring 142 can also resiliently connect the plunger 108 and the frame 110 to one another. Through this connection, the first spring 142 can urge the frame 110 to move in the first direction 134 relative to the plunger 108. Accordingly, the first spring 142 can resist movement of the plunger 108 in the first direction 134 relative to the frame 110 while the frame 110 is in contact with the target surface 132. In at least some cases, the first spring 142 is under compression within the housing 106 when the deployment device 102 is in its default state before use.

The deployment device 102 can further include a sleeve 144 and a second spring 146 that work together to retract the needle 126 from the skin 130 after the carrier 138 moves the sensor 122 into contact with the target surface 132. The deployment device 102 can also include a cartridge 148 and a third spring 150 that work together to safely encase the needle 126 after the needle 126 is retracted from the skin 130. Movement of the sensor 122, the needle 126, and the cannula 127 in the first direction 134 via action of the first spring 142, retraction of the needle 126 via action of the second spring 146, and encasement of the needle 126 in the cartridge 148 via action of the third spring 150 can occur in a cascade in which the preceding process triggers the subsequent process. After this cascade of processes, the needle 126 can be encased in the cartridge 148 and the carrier 138 and the retainer 140 can be in an extended position. With reference to FIGS. 2 and 4 together, the extended position can be one in which the carrier 138 and the retainer 140 are at the deployment window 120. Certain relevant details, including details regarding encasement of the needle 126 in the cartridge 148, are further discussed in U.S. Patent Application No. 17/072,867, published as US20220117627, which in incorporated herein by reference in its entirety. To the extent this reference conflicts with the present disclosure, the present disclosure controls.

FIGS. 5 and 6 are perspective views of the cap 116 and the frame 110 respectively. With reference to FIGS. 4-6 together, threads 152 on an outer surface of the plunger 108 and complementary threads 154 on an inner surface of the cap 116 can be engaged to releasably connect the plunger 108 and the cap 116 to one another. When in place, the cap 116 can confine the first spring 142, the second spring 146, the third spring 150, the needle 126, and the cartridge 148 within the housing 106. In at least some embodiments, the first spring 142, the second spring 146, and the third spring 150 are metal, whereas the cap 116, the plunger 108, the frame 110, the sleeve 144, the carrier 138, and the retainer 140 are plastic. In these cases, it can be useful to separate the metal components from the plastic components for recycling by different processes. Moreover, after use, the needle 126 is hazardous medical waste. The needle 126, therefore, typically must be disposed of rather than recycled. Certain features of the deployment device 102 can facilitate safely separating the metal, plastic, and hazardous components from one another. For example, when the cap 116 is removed, the first spring 142, the second spring 146, the third spring 150, and the needle 126 (within the cartridge 148) can be removable from the housing 106. For example, an end user of the deployment device 102 can remove the cap 116 and tilt and/or shake the remaining portion of the deployment device 102 until the removable components separate from the other components. In some cases, the removable components fall away from the other components by gravity alone with no need for shaking. In other cases, some shaking may be needed to dislodge these components. In still other cases, the springs may push other components apart and/or out of the housing 106 if some residual compression remains in one or all of the springs.

It can be useful to reduce or eliminate the possibility of an end user unintentionally opening the housing 106 before the deployment device 102 is used. Opening the housing 106 before the deployment device 102 is used may cause the deployment device 102 to malfunction, may cause the deployment device 102 to be difficult to reassemble, may cause the first spring 142 to eject forcefully, and/or may have one or more other undesirable effects. For this and/or other reasons, the deployment device 102 can include one or more stops 156 (individually identified as stops 156a, 156b) that, when engaged, are configured to block separation of the cap 116 from the plunger 108. As shown in FIG. 6, the stops 156 can be carried by the frame 110. For example, the stops 156 can be part of a rim 158 at an uppermost portion of the frame 110. In at least some cases, the rim 158 defines gaps 160 (individually identified as gaps 160a, 160b) circumferentially adjacent to the stops 156. The cap 116 can include one or more features configured to interact with the stops 156. As shown in FIG. 5, the cap 116 can include a column 162 and supports 164 (individually identified as supports 164a, 164b) circumferentially spaced apart from one another and extending radially outward from the column 162. With reference to FIGS. 3A-6 together, the cap 116 can further include projections 166 (individually identified as projections 166a, 166b) extending outwardly from the supports 164a, 164b respectively, in a plane perpendicular to the first and second directions 134, 136.

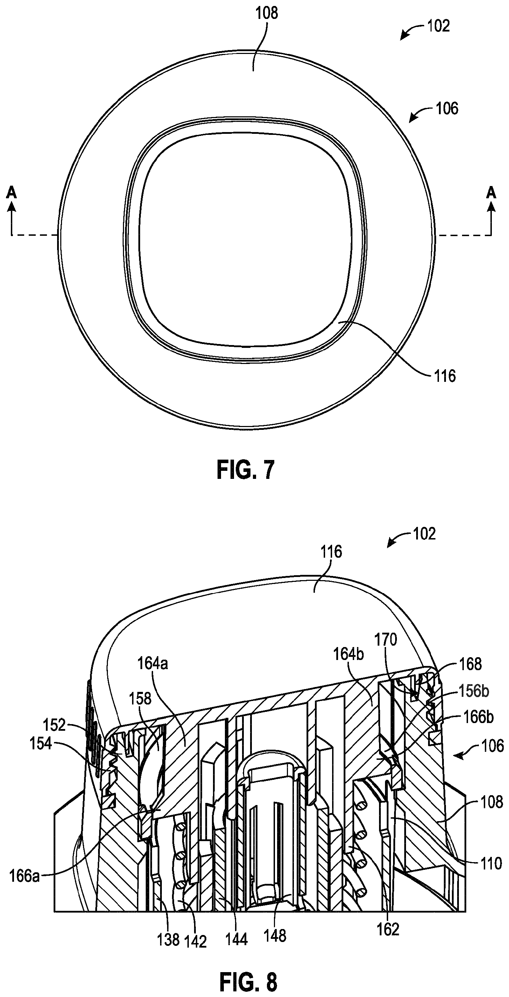

FIG. 7 is a top plan view of the deployment device 102. FIG. 8 is a cross-sectional perspective view of a portion of the deployment device 102 taken along the line A-A in FIG. 7. In FIG. 8, the deployment device 102 is shown in a default state before use. In this state, the stops 156 can be engaged by default due to the default position of the frame 110 relative to the cap 116. With reference now to FIGS. 3A-8 together, the stops 156a, 156b, when engaged, can be circumferentially adjacent to the projections 166a, 166b, respectively, along a circle within the plane perpendicular to the first and second directions 134, 136. Correspondingly, the projections 166a, 166b can protrude radially into the gaps 160a, 160b. The stops 156, when engaged, can be configured to block rotation of the cap 116 relative to the plunger 108 by the projections 166 being engaged with the gaps 160 thereby preventing rotation. The stops 156 can be configured to disengage at least partially in response to the frame 110 moving in the second direction 136 relative to the plunger 108, thereby enabling the projections 166 to linearly translate away from the gaps 160 and below the stops 156. For example, the stops 156 can be engaged by default when the deployment device 102 is in the states shown in FIGS. 3A and 3D and disengaged when the deployment device 102 is in the states shown in FIGS. 3B and 3C. An end user is unlikely to unintentionally remove the cap 116 from the plunger 108 when the deployment device 102 is in the states shown in FIGS. 3B and 3C because these are transient states. After the deployment device 102 is used (FIG. 3D) an end user can intentionally shift the frame 110 in the second direction 136 relative to plunger 108 to hold the stops 156 in a disengaged state and simultaneously unscrew the cap 116.

In at least some cases, the deployment device 102 includes one or more features that help to maintain a sterile environment within the housing 106 before use. As shown in FIG. 8, the cap 116 can include an annular flange 168 configured to resiliently engage an annular sealing surface 170 of the plunger 108 when the cap 116 and the plunger 108 are connected to one another. Alternatively, a counterpart of the plunger 108 can include a counterpart of the annular flange 168 and a counterpart of the cap 116 can include a counterpart of the annular sealing surface 170. Other types of sealing structures are also possible.

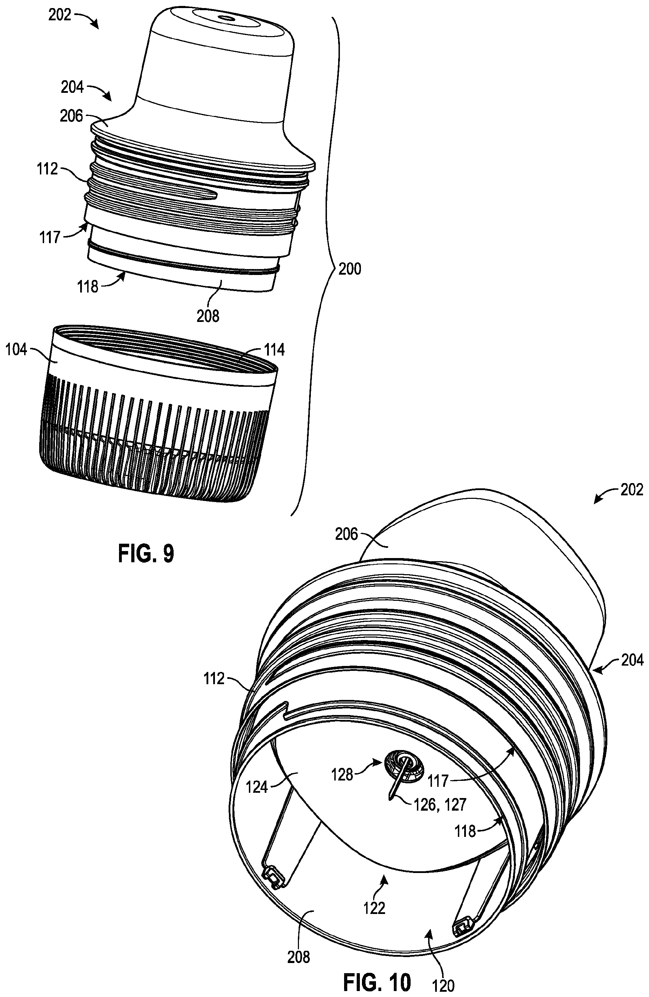

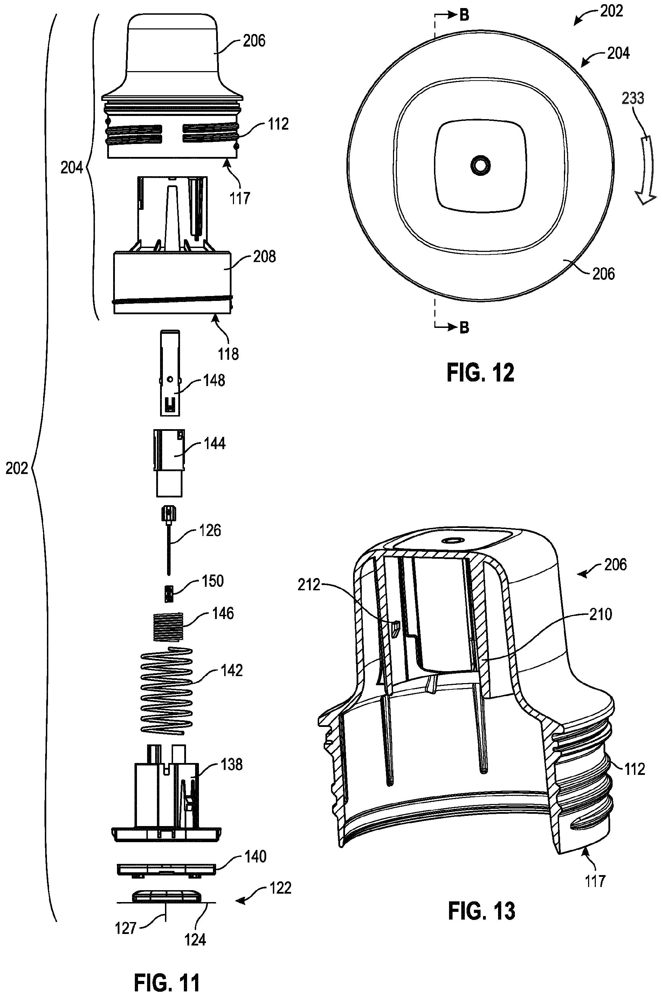

FIG. 9 is an exploded perspective view of another system 200 including a deployment device 202 in accordance with at least some embodiments of the present technology. FIGS. 10 and 11 are a perspective view and an exploded perspective view, respectively, of the deployment device 202. The deployment device 202 can be similar to the deployment device 102 described above with reference to the deployment device 102, but with different opening and locking features. For example, the deployment device 202 can include a housing 204 including a plunger 206 and a frame 208 configured to separate from one another to open the housing 204. Accordingly, the plunger 206 can be without a counterpart of the cap 116 described above with reference to the deployment device 102.

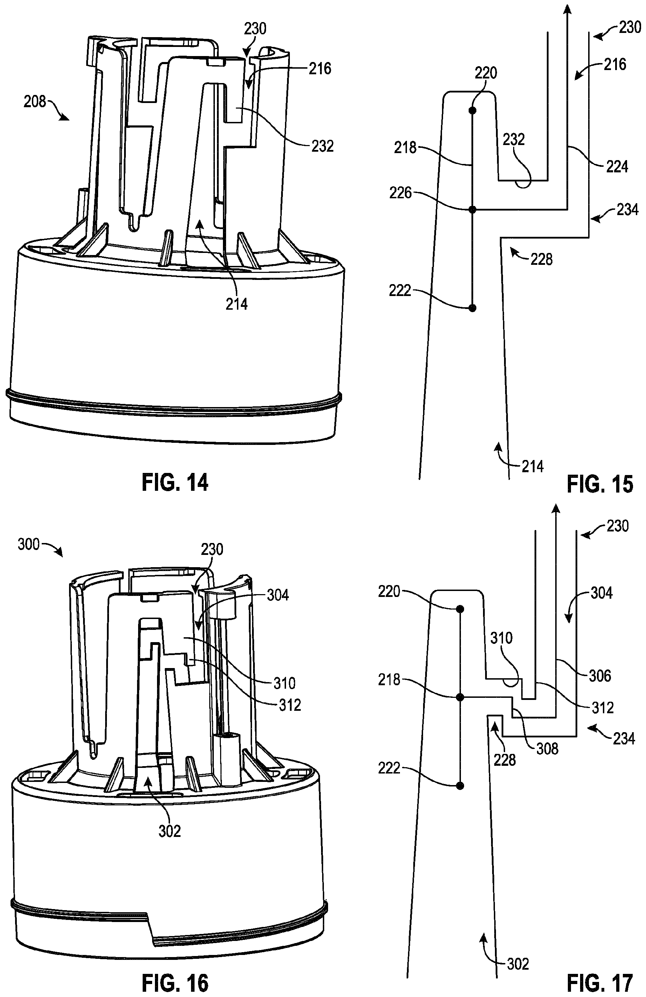

FIG. 12 is a top plan view of the plunger 206. FIG. 13 is a cross-sectional perspective view of the plunger 206 taken along the line B-B in FIG. 12. FIG. 14 is a perspective view of the frame 208. With reference to FIGS. 12-14 together, the plunger 206 and the frame 208 can include features that work together to cause the housing 204 to be openable by a deliberate, multi-part movement of the frame 208 relative to the plunger 206. For example, the plunger 206 can include an annular flange 210 centrally positioned and extending downwardly within the housing 204 from an uppermost portion of the plunger 206. The plunger 206 can further include a key 212 projecting inwardly from the annular flange 210. The frame 208 can define a first channel 214 and a second channel 216. FIG. 15 is a schematic illustration of the first and second channels 214, 216. As shown in FIG. 15, the first channel 214 can extend along a first path 218 parallel to the first and second directions 134, 136 (FIG. 3B). When the plunger 206 and the frame 208 are connected to one another, the key 212 can extend into the first channel 214 in a plane perpendicular to the first path 218. The frame 208 can define a range of motion in the second direction 136 relative to the plunger 206. Furthermore, moving the frame 208 in the second direction 136 relative to the plunger 206 through the full range of motion can cause the key 212 to move within the first channel 214 between a first end 220 of the first path 218 and an opposite second end 222 of the first path 218. The first channel 214 can be closed ended at the first end 220 of the first path 218 such that a portion of the frame 208 above the first channel 214 interacts with the key 212 to block movement of the frame 208 in the first direction 134 relative to the plunger 206 beyond the range of motion when the deployment device 202 is in a default state.

The second channel 216 can extend along a second path 224 that branches from the first path 218 at a branch point 226 between the first and second ends 220, 222 of the first path 218. The second channel 216 can be a through channel. For example, the second channel 216 can define a mouth 228 at the branch point 226 and an open end 230 opposite the branch point 226. In at least some cases, the mouth 228 is spaced apart from the first end 220 of the first path 218 by at least 10% of a length of the first path 218 and is spaced apart from the second end 222 of the first path 218 by at least 10% of the length of the first path 218. In these and other cases, the frame 208 can carry a stop 232 of the deployment device 202 that, when engaged, is configured to block separation of the frame 208 from the plunger 206. For example, the stop 232 can be circumferentially adjacent to a portion of the first channel 214 at the first end 220 of the first path 218 such that interaction between the stop 232 and the key 212 blocks rotation of the frame 208 relative to the plunger 206 when the deployment device 202 is in the default state.

The stop 232 can be configured to disengage at least partially in response to the frame 208 first moving in the second direction 136 relative to the plunger 206 while the key 212 moves along a portion of the first path 218 beginning at the first end 220 of the first path 218 and extending to the branch point 226. The stop 232 can be configured to disengage further in response to the frame 208 then rotating in a third direction 233 relative to the plunger 206 perpendicular to the first and second directions 134, 136 while the key 212 moves along a portion of the second path 224 beginning at the branch point 226 and extending to a turn 234 in the second path 224. During this movement, the stop 232 can interact with the key 212 to block movement of the frame 208 in the first direction 134 relative to the plunger 206 rather than rotation of the frame 208 relative to the plunger 206. After this movement, the frame 208 can be configured to separate from the plunger 206 and thereby open the housing 204 at least partially in response to moving in the first direction 134 relative to the plunger 206 while the key 212 moves along a portion of the second path 224 beginning at the turn 234 and extending through the open end 230. Before separating from the plunger 206, the frame 208 can confine the first spring 142 within the housing 204. After the frame 208 and the plunger 206 are separated from one another, the first spring 142 can become removable from the housing 204, such as by shaking, gravity, and/or residual spring force pushing the components apart.

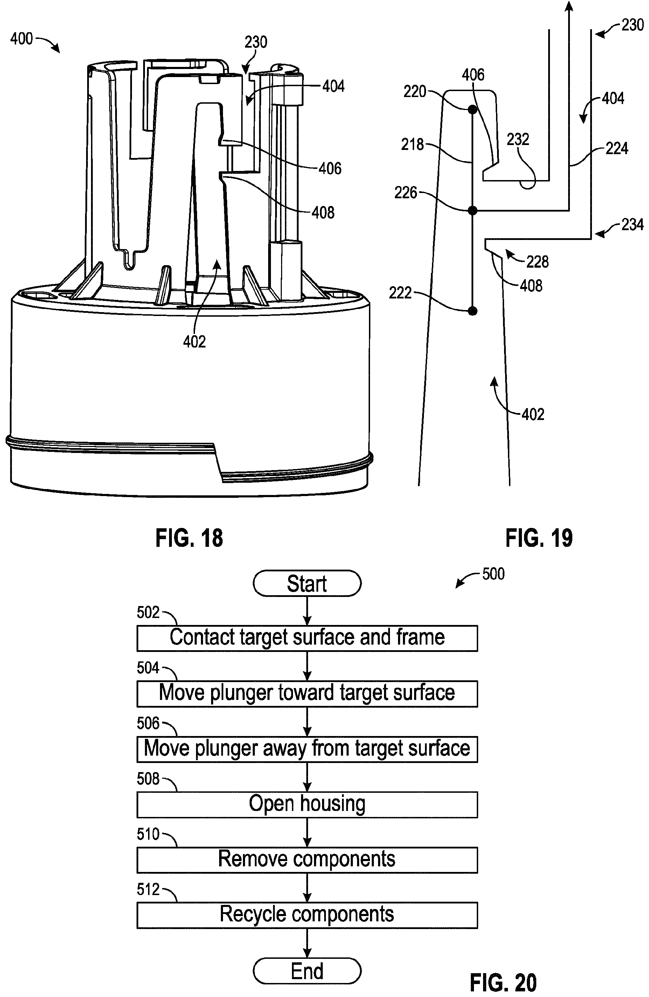

Although an end user is unlikely to perform the multi-part movement of the frame 208 relative to the plunger 206 described above, counterparts of the frame 208 can include additional features to further reduce this possibility. FIGS. 16-20 illustrate two such counterparts. In particular, FIG. 16 is a perspective view of a frame 300 of a housing of another deployment device in accordance with at least some embodiments of the present technology. FIG. 17 is a schematic illustration of a first channel 302 and a second channel 304 defined by the frame 300. As shown in FIGS. 16 and 17, the second channel 304 can extend along a second path 306 that includes a step 308. The frame 300 can include a stop 310 that includes a tab 312 at the step 308. Navigating through the step 308 (e.g., by moving the key 212 past the tab 312) can add another level of intentionality to manipulation of the frame 300 relative to a counterpart of the plungers 108, 206 to open a counterpart of the housings 106, 204. FIG. 18 is a perspective view of a frame 400 of a housing of yet another deployment device in accordance with at least some embodiments of the present technology. FIG. 19 is a schematic illustration of a first channel 402 and a second channel 404 defined by the frame 400. As shown in FIGS. 18 and 19, the frame 400 can include a first nub 406 at a first side of the mouth 228 in a dimension parallel to the first path 218 and a second nub 408 at an opposite second side of the mouth 228 in the dimension parallel to the first path 218. One or both of the first and second nubs 406, 408 can be configured to guide the key 212 (FIG. 13) away from the mouth 228 while the key 212 moves from the first end 220 of the first path 218 to the second end 222 of the first path 218. In addition or alternatively, one or both of the first and second nubs 406, 408 can be configured to guide the key 212 (FIG. 13) away from the mouth 228 while the key 212 moves from the second end 222 of the first path 218 to the first end 220 of the first path 218.

FIG. 20 is a block diagram illustrating a method 500 for connecting a transcutaneous device to skin of a subject in accordance with at least some embodiments of the present technology. For simplicity, aspects of the method 500 will be described primarily in the context one of the deployment devices 102, 202, the sensor 122, and/or the alternative frames 300, 400 described herein. It should be understood, however, that the method 500, when suitable, and/or portions of the method 500, when suitable, can be practiced with respect to other deployment devices, transcutaneous devices, frames, etc. described herein and not described herein. With reference to Figures 1-20 together, the method 500 can include contacting the target surface 132 and the frame 110, 208, 300, 400 (block 502). In at least some cases, this occurs while the sensor 122 is staged within the housing 106, 204, while the needle 126 is staged within the housing 106, 204, and/or while the housing 106, 204 is locked. The housing 106 can be locked, for example, while the stops 156 block rotation of the cap 116 relative to the plunger 108. The housing 204 can be locked, for example, while the stop 232 blocks rotation of the frame 208 relative to the plunger 206. Next, the method 500 can include moving the plunger 108, 206 toward the target surface 132 in the first direction 134 relative to the frame 110, 208, 300, 400 (block 504). This can occur while the frame 110, 208, 300, 400 is in contact with the target surface 132 and/or while the frame 110, 208, 300, 400 moves in the second direction 136 relative to the plunger 108, 206. Furthermore, this can occur while the key 212 moves along the first path 218 within the first channel 214, 302, 402. The relative movement of the frame 110, 208, 300, 400 and the plunger 108, 206 can at least partially cause the sensor 122 to move into contact with the target surface 132. In addition or alternatively, the relative movement of the frame 110, 208, 300, 400 and the plunger 108, 206 can at least partially cause the needle 126 to move toward and to pierce the target surface 132 and the skin 130, withdraw from the skin 130, move away from the target surface 132, and move into the cartridge 148. These actions can occur, for example, via a cascade of triggers.

The method 500 can further include moving the plunger 108, 206 away from the target surface 132 (block 506). This can occur after the sensor 122 moves into contact with the target surface 132, while the first spring 142 moves the frame 110, 208, 300, 400 in the first direction 134 relative to the plunger 108, 206, and/or while the key 212 moves along the first path 218 within the first channel 214, 302, 402. At this point, the deployment device 102, 202 can be in a used state and ready for disassembly and recycling. The disassembly process can include moving the frame 110, 208, 300, 400 in the second direction 136 relative to the plunger 108, 206 after moving the plunger 108, 206 away from the target surface 132. This can at least partially cause the housing 106, 204 to unlock, such as by disengaging the stops 156 or partially disengaging the stop 232. In the context of the deployment device 202, unlocking the housing 204 can further include blindly aligning (e.g., via tactile feedback) the key 212 with the mouth 228 while moving the frame 208, 300, 400 in the second direction 136 relative to the plunger 206 after moving the plunger 206 away from the target surface 132. In these and other cases, unlocking the housing 204 can still further include rotating the frame 208, 300, 400 in the third direction perpendicular to the first and second directions 134, 136 relative to the plunger 206 after moving the frame 208, 300, 400 in the second direction 136 relative to the plunger 206 and while the key 212 moves along the second path 224, 306 within the second channel 216, 304, 404.

After the housing 106, 204 is unlocked, the method 500 can include opening the housing 106, 204 (block 508). With respect to the housing 106, the opening process can include separating the cap 116 from the plunger 108, such as by rotating the cap 116 relative to the plunger 108. With respect to the housing 204, the opening process can include separating the frame 208, 300, 400 from the plunger 206 after rotating the frame 208, 300, 400 in the third direction relative to the plunger 206. Separating the frame 208, 300, 400 from the plunger 206 can include moving the frame 208, 300, 400 in the first direction 134 relative to the plunger 206. After opening the housing 106, 204, the method 500 can include removing components from the housing 106, 204 (block 510) and then recycling at least some of the removed and/or remaining components (block 512). Removing the components can include allowing the components to drop from the housing 106, 204 by gravity. In some cases, removing the components further includes tilting and/or shaking the housing 106, 204 to facilitate this separation. The removed components can include some or all components that are not co-recyclable with the housing 106, 204, such as because the removed components are metal and/or hazardous and the housing 106, 204 is plastic. The removed components can include the first spring 142, the second spring 146, the third spring 150, and the needle 126, which can all be metal. The removed components can further include the cartridge 148 which may be plastic, but is useful to make disposal of the needle 126 less hazardous.

This disclosure is not intended to be exhaustive or to limit the present technology to the precise forms disclosed herein. Although specific embodiments are disclosed herein for illustrative purposes, various equivalent modifications are possible without deviating from the present technology, as those of ordinary skill in the relevant art will recognize. In some cases, well-known structures and functions have not been shown or described in detail to avoid unnecessarily obscuring the description of the embodiments of the present technology. Although steps of methods may be presented herein in a particular order, in alternative embodiments the steps may have another suitable order. Similarly, certain aspects of the present technology disclosed in the context of particular embodiments can be combined or eliminated in other embodiments. Furthermore, while advantages associated with certain embodiments may be disclosed herein in the context of those embodiments, other embodiments may also exhibit such advantages, and not all embodiments need necessarily exhibit such advantages or other advantages disclosed herein to fall within the scope of the present technology. This disclosure and the associated technology can encompass other embodiments not expressly shown or described herein.

Throughout this disclosure, the singular terms "a," "an," and "the" include plural referents unless the context clearly indicates otherwise. Similarly, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the terms "comprising," "including," and the like are used throughout this disclosure to mean including at least the recited feature(s) such that any greater number of the same feature(s) and/or one or more additional types of features are not precluded. Directional terms, such as "upper," "lower," "front," "back," "vertical," and "horizontal," may be used herein to express and clarify the relationship between various structures. It should be understood that such terms do not denote absolute orientation. Furthermore, reference herein to "one embodiment," "an embodiment," or similar phrases means that a particular feature, structure, operation, or characteristic described in connection with such phrases can be included in at least one embodiment of the present technology. Thus, such phrases as used herein are not necessarily all referring to the same embodiment. Finally, it should be noted that various particular features, structures, operations, and characteristics of the embodiments described herein may be combined in any suitable manner in additional embodiments in accordance with the present technology.

## Claims

1. A deployment device (102, 202), comprising:
a housing (106, 204) including:
a plunger (108, 206),
a frame (110, 208, 300, 400) movably connected to the plunger and defining a deployment window (120), and
a cap (116) releasably connected to the plunger;
a spring (142) confined within the housing by the cap, wherein the plunger and the frame are resiliently connected to one another via the spring, and wherein the spring urges the frame to move in a first direction (134) relative to the plunger;
a carrier (138) movably connected to the frame, wherein the carrier is configured to move a transcutaneous device into contact with a target surface (132) of a subject via the deployment window at least partially in response to the frame moving in a second direction (136) relative to the plunger, the second direction being opposite the first direction; and
a stop (156) that, when engaged, is configured to block separation of the cap from the plunger, wherein the stop is configured to disengage at least partially in response to the frame moving in the second direction relative to the plunger.

2. The deployment device of claim 1, wherein the frame carries the stop,
and/or
wherein:
one of the plunger and the cap includes an annular sealing surface (170); and
the other of the plunger and the cap includes an annular flange (168) configured to resiliently engage the sealing surface when the cap is connected to the plunger,
and/or
wherein the spring is removable from the housing when the cap is separated from the plunger.

3. The deployment device of any of claims 1-2, wherein:
the cap and the plunger include complementary threads (114) through which the cap and the plunger are releasably connected to one another; and
the stop, when engaged, is configured to block rotation of the cap relative to the plunger
and/or
wherein:
the cap includes a support (164) and a projection (166) extending outwardly from the support in a plane perpendicular to the first and second directions; and
the stop, when engaged, is circumferentially adjacent to the projection along a circle within the plane.

4. A deployment device (202), comprising:
a housing (204) including:
a plunger (206), and
a frame (208, 300, 400) movably connected to the plunger and defining a deployment window (120),
a spring (142) confined within the housing by the frame, wherein the plunger and the frame are resiliently connected to one another via the spring, and wherein the spring urges the frame to move in a first direction (134) relative to the plunger;
a carrier (138) movably connected to the frame, wherein the carrier is configured to move a transcutaneous device into contact with a target surface (132) of a subject via the deployment window at least partially in response to the frame moving in a second direction (136) relative to the plunger, the second direction being opposite the first direction; and
a stop (232) that, when engaged, is configured block separation of the frame from the plunger, wherein the stop is configured to disengage at least partially in response to the frame moving in the second direction relative to the plunger and then rotating in a third direction (233) relative to the plunger, the third direction being perpendicular to the first and second directions,
particularly
wherein the frame is configured to separate from the plunger at least partially in response to the frame moving in the first direction relative to the plunger after rotating in the third direction relative to the plunger.

5. The deployment device of claim 4, wherein:
the frame defines a channel (214, 216, 302, 304, 402, 404) extending along a path (218, 224) parallel to the first and second directions;
the plunger includes a key (212) extending into the channel in a plane perpendicular to the path;
the carrier is configured to move the transcutaneous device into contact with the target surface via the deployment window at least partially in response to the frame moving in the second direction relative to the plunger;
the frame defines a range of motion in the second direction relative to the plunger; and
moving the frame in the second direction relative to the plunger through the full range of motion at least partially causes the key to move within the channel between a first end (220) of the path and an opposite second end (222) of the path.

6. The deployment device of claim 5, wherein:
the channel and the path are a first channel (214, 302, 402) and a first path (218), respectively; and
the frame defines a second channel (216, 304, 404) extending along a second path (224) that branches from the first path at a branch point (226) between the first and second ends of the first path.

7. The deployment device of claim 6, wherein the stop is configured to disengage at least partially in response to the frame moving in the second direction relative to the plunger while the key moves along a portion of the first path beginning at the first end of the first path, and the frame then moving in the third direction relative to the plunger while the key moves along a portion of the second path beginning at the branch point,
particularly
wherein:
the first channel is closed ended at the first end of the first path; and
the second channel is a through channel.

8. The deployment device of any of claims 6-7, wherein the second path includes a step (308),
and/or
wherein:
the second channel includes a mouth (228) at the branch point;
the mouth is spaced apart from the first end of the first path by at least 10% of a length of the first path; and
the mouth is spaced apart from the second end of the first path by at least 10% of the length of the first path.

9. The deployment device of any of claims 6-8, wherein:
the second channel includes a mouth at the branch point; and
the frame includes:
a first nub (406) at a first side of the mouth in a dimension parallel to the first path, and
a second nub (408) at an opposite second side of the mouth in the dimension parallel to the first path,
particularly
wherein the first and second nubs are configured to guide the key away from the mouth while the key moves from the first end of the first path to the second end of the first path, while the key moves from the second end of the first path to the first end of the first path, or both.

10. A method for connecting a transcutaneous device to skin of a subject, the method comprising:
contacting a target surface (132) of the subject and a frame (110, 208, 300, 400) of a housing of a deployment device (102, 202) while the transcutaneous device is staged within the housing and while the housing is locked;
moving a plunger (108, 206) of the housing toward the target surface in a first direction (134) relative to the frame while the frame is in contact with the target surface and while the frame moves in a second direction (136) relative to the plunger, the second direction being opposite the first direction, wherein moving the plunger toward the target surface in the first direction relative to the frame while the frame is in contact with the target surface at least partially causes the transcutaneous device to move into contact with the target surface;
moving the plunger away from the target surface after the transcutaneous device moves into contact with the target surface and while a spring (142) of the deployment device moves the frame in the first direction relative to the plunger;
moving the frame in the second direction relative to the plunger after moving the plunger away from the target surface, wherein moving the frame in the second direction relative to the plunger after moving the plunger away from the target surface at least partially causes the housing to unlock;
opening the housing after the housing unlocks; and
removing the spring from the housing after opening the housing.

11. The method of claim 10, wherein:
opening the housing includes rotating a cap (116) of the housing relative to the plunger;
contacting the target surface and the frame includes contacting the target surface and the frame while a stop (156) of the deployment device blocks rotation of the cap relative to the plunger; and
moving the frame in the second direction relative to the plunger after moving the plunger away from the target surface at least partially causes the housing to unlock by disengaging the stop
and/or
wherein removing the spring includes tilting the housing to allow the spring to drop from the housing by gravity.

12. The method of any of claims 10-11, wherein:
contacting the target surface and the frame includes contacting the target surface and the frame while a needle (126) of the deployment device is staged within the housing; and
moving the plunger toward the target surface in the first direction relative to the frame while the frame is in contact with the target surface at least partially causes the needle to move toward and to pierce the target surface and the skin,
particularly
wherein:
moving the plunger toward the target surface in the first direction relative to the frame while the frame is in contact with the target surface at least partially causes the needle to withdraw from the skin, to move away from the target surface, and to move into a cartridge within the housing; and
the method further comprises removing the needle and the cartridge from the housing after opening the housing.

13. A method for connecting a transcutaneous device to skin of a subject, the method comprising:
contacting a target surface (132) of the subject and a frame (110, 208, 300, 400) of a housing of a deployment device while the transcutaneous device is staged within the housing;
moving a plunger (108, 206) of the deployment device toward the target surface in a first direction (134) relative to the frame while the frame is in contact with the target surface and while the frame moves in a second direction (136) relative to the plunger, the second direction being opposite the first direction, wherein moving the plunger toward the target surface in the first direction relative to the frame while the frame is in contact with the target surface at least partially causes the transcutaneous device to move into contact with the target surface;
moving the plunger away from the target surface after the transcutaneous device moves into contact with the target surface and while a spring (142) of the deployment device moves the frame in the first direction relative to the plunger;
moving the frame in the second direction relative to the plunger after moving the plunger away from the target surface;
rotating the frame in a third direction (233) relative to the plunger after moving the frame in the second direction relative to the plunger, the third direction being perpendicular to the first and second directions;
separating the frame from the plunger after rotating the frame in the third direction relative to the plunger; and
removing the spring from the housing after separating the frame from the plunger.

14. The method of claim 13, further comprising moving the frame in the first direction relative to the plunger while separating the frame from the plunger,
and/or
wherein removing the spring includes allowing the spring to drop from the housing by gravity.

15. The method of any of claims 13-14, wherein moving the plunger toward the target surface in a first direction relative to the frame while the frame is in contact with the target surface includes moving the plunger toward the target surface in a first direction relative to the frame while the frame is in contact with the target surface and while a key (212) of the plunger moves along a path (218, 224) within a channel (214, 216, 302, 304, 402, 404) defined by the frame,
particularly
wherein:
the channel and the path are a first channel (214, 302, 402) and a first path (218), respectively;
moving the plunger away from the target surface after the transcutaneous device moves into contact with the target surface includes moving the plunger away from the target surface after the transcutaneous device moves into contact with the target surface and while the key moves along the first path within the first channel;
rotating the frame in the third direction relative to the plunger after moving the frame in the second direction relative to the plunger includes rotating the frame in the third direction relative to the plunger after moving the frame in the second direction relative to the plunger and while the key moves along a second path (224) within a second channel (216, 304, 404) defined by the frame; and
the second path branches from the first path,
particularly
further comprising blindly aligning the key with a mouth (228) of the second channel while moving the frame in the second direction relative to the plunger after moving the plunger away from the target surface.

## Patentansprüche

1. Einsatzvorrichtung (102, 202), umfassend:
ein Gehäuse (106, 204), das einschließt:
einen Kolben (108, 206),
einen Rahmen (110, 208, 300, 400), der mit dem Kolben bewegbar verbunden ist und ein Einsatzfenster (120) definiert, und
eine Kappe (116), die mit dem Kolben lösbar verbunden ist;
eine Feder (142), die durch die Kappe innerhalb des Gehäuses begrenzt ist, wobei der Kolben und der Rahmen über die Feder elastisch miteinander verbunden sind und wobei die Feder den Rahmen drängt, sich in eine erste Richtung (134) relativ zu dem Kolben zu bewegen;
einen Träger (138), der mit dem Rahmen bewegbar verbunden ist, wobei der Träger konfiguriert ist, um eine transkutane Vorrichtung über das Einsatzfenster in Kontakt mit einer Zieloberfläche (132) eines Subjekts zu bewegen, mindestens teilweise als Reaktion auf den Rahmen, der sich in eine zweite Richtung (136) relativ zu dem Kolben bewegt, wobei die zweite Richtung der ersten Richtung gegenüberliegt; und
einen Anschlag (156), der, wenn er in Eingriff steht, konfiguriert ist, um eine Trennung der Kappe von dem Kolben zu blockieren, wobei der Anschlag konfiguriert ist, um sich mindestens teilweise als Reaktion auf den Rahmen zu lösen, der sich in die zweite Richtung relativ zu dem Kolben bewegt.

2. Einsatzvorrichtung nach Anspruch 1, wobei der Rahmen den Anschlag trägt,
und/oder
wobei:
einer von dem Kolben und der Kappe eine ringförmige Dichtungsoberfläche (170) einschließt; und
der andere Teil von dem Kolben und der Kappe einen ringförmigen Flansch (168) einschließt, der konfiguriert ist, um die Dichtungsoberfläche elastisch in Eingriff zu nehmen, wenn die Kappe mit dem Kolben verbunden ist,
und/oder
wobei die Feder aus dem Gehäuse entfernt werden kann, wenn die Kappe von dem Kolben getrennt wird.

3. Einsatzvorrichtung nach einem der Ansprüche 1 bis 2, wobei:
die Kappe und der Kolben komplementäre Gewinde (114) einschließen, durch die hindurch die Kappe und der Kolben miteinander lösbar verbunden sind; und
der Anschlag, wenn er in Eingriff steht, konfiguriert ist, um eine Drehung der Kappe relativ zu dem Kolben zu blockieren
und/oder
wobei:
die Kappe eine Stütze (164) und einen Vorsprung (166) einschließt, der sich von der Stütze in einer Ebene senkrecht zu der ersten und der zweiten Richtung nach außen erstreckt; und
der Anschlag, wenn er in Eingriff steht, in Umfangsrichtung an den Vorsprung entlang eines Kreises innerhalb der Ebene angrenzt.

4. Einsatzvorrichtung (202), umfassend:
ein Gehäuse (204), das einschließt:
einen Kolben (206) und
einen Rahmen (208, 300, 400), der mit dem Kolben bewegbar verbunden ist und ein Einsatzfenster (120) definiert,
eine Feder (142), die durch den Rahmen innerhalb des Gehäuses begrenzt ist, wobei der Kolben und der Rahmen über die Feder elastisch miteinander verbunden sind und wobei die Feder den Rahmen drängt, sich in eine erste Richtung (134) relativ zu dem Kolben zu bewegen;
einen Träger (138), der mit dem Rahmen bewegbar verbunden ist, wobei der Träger konfiguriert ist, um eine transkutane Vorrichtung über das Einsatzfenster in Kontakt mit einer Zieloberfläche (132) eines Subjekts zu bewegen, mindestens teilweise als Reaktion auf den Rahmen, der sich in eine zweite Richtung (136) relativ zu dem Kolben bewegt, wobei die zweite Richtung der ersten Richtung gegenüberliegt; und
einen Anschlag (232), der, wenn er in Eingriff steht, konfiguriert ist, um die Trennung des Rahmens von dem Kolben zu blockieren, wobei der Anschlag konfiguriert ist, um sich mindestens teilweise als Reaktion auf den Rahmen zu lösen, der sich in die zweite Richtung relativ zu dem Kolben bewegt und sich dann in eine dritte Richtung (233) relativ zu dem Kolben dreht, wobei die dritte Richtung senkrecht zu der ersten und der zweiten Richtung verläuft,
insbesondere
wobei der Rahmen konfiguriert ist, um sich von dem Kolben mindestens teilweise als Reaktion auf den Rahmen zu trennen, der sich in die erste Richtung relativ zu dem Kolben bewegt, nachdem er sich in die dritte Richtung relativ zu dem Kolben gedreht hat.

5. Einsatzvorrichtung nach Anspruch 4, wobei:
der Rahmen einen Kanal (214, 216, 302, 304, 402, 404) definiert, der sich entlang eines Pfads (218, 224) parallel zu der ersten und der zweiten Richtung erstreckt;
der Kolben einen Keil (212) einschließt, der sich in einer Ebene senkrecht zu dem Pfad in den Kanal hinein erstreckt;
der Träger konfiguriert ist, um die transkutane Vorrichtung über das Einsatzfenster in Kontakt mit der Zieloberfläche zu bringen, mindestens teilweise als Reaktion auf den Rahmen, der sich in die zweite Richtung relativ zu dem Kolben bewegt;
der Rahmen einen Bewegungsbereich in der zweiten Richtung relativ zu dem Kolben definiert; und
das Bewegen des Rahmens in der zweiten Richtung relativ zu dem Kolben durch den gesamten Bewegungsbereich hindurch den Keil mindestens teilweise veranlasst, sich innerhalb des Kanals zwischen einem ersten Ende (220) des Pfads und einem gegenüberliegenden zweiten Ende (222) des Pfads zu bewegen.

6. Einsatzvorrichtung nach Anspruch 5, wobei:
der Kanal und der Pfad ein erster Kanal (214, 302, 402) beziehungsweise ein erster Pfad (218) sind; und
der Rahmen einen zweiten Kanal (216, 304, 404) definiert, der sich entlang eines zweiten Pfads (224) erstreckt, der an einem Verzweigungspunkt (226) zwischen dem ersten und dem zweiten Ende des ersten Pfads von dem ersten Pfad abzweigt.

7. Einsatzvorrichtung nach Anspruch 6, wobei der Anschlag konfiguriert ist, um sich mindestens teilweise als Reaktion auf den Rahmen zu lösen, der sich in die zweite Richtung relativ zu dem Kolben bewegt, während sich der Keil entlang eines Abschnitts des ersten Pfads bewegt, der an dem ersten Ende des ersten Pfads beginnt, und sich der Rahmen dann in die dritte Richtung relativ zu dem Kolben bewegt, während sich der Keil entlang eines Abschnitts des zweiten Pfads bewegt, der an dem Verzweigungspunkt beginnt.
insbesondere
wobei:
der erste Kanal an dem ersten Ende des ersten Pfads endverschlossen ist; und
der zweite Kanal ein Durchgangskanal ist.

8. Einsatzvorrichtung nach einem der Ansprüche 6 bis 7, wobei der zweite Pfad einen Schritt (308) einschließt.
und/oder
wobei:
der zweite Kanal an dem Verzweigungspunkt eine Mündung (228) einschließt;
die Mündung von dem ersten Ende des ersten Pfads um mindestens 10 % einer Länge des ersten Pfads beabstandet ist; und
die Mündung von dem zweiten Ende des ersten Pfads um mindestens 10 % der Länge des ersten Pfads beabstandet ist.

9. Einsatzrichtung nach einem der Ansprüche 6 bis 8, wobei:
der zweite Kanal eine Mündung an dem Verzweigungspunkt einschließt;
und
der Rahmen einschließt:
einen ersten Noppen (406) an einer ersten Seite der Mündung in einer Abmessung parallel zu dem ersten Pfad, und
einen zweiten Noppen (408) an einer gegenüberliegenden zweiten Seite der Mündung in der Abmessung parallel zu dem ersten Pfad,
insbesondere
wobei der erste und der zweite Noppen konfiguriert sind, um den Keil von der Mündung weg zu führen, während sich der Keil von dem ersten Ende des ersten Pfads zu dem zweiten Ende des ersten Pfads bewegt, während sich der Keil von dem zweiten Ende des ersten Pfads zu dem ersten Ende des ersten Pfads bewegt, oder beides.

10. Verfahren zum Verbinden einer transkutanen Vorrichtung mit der Haut eines Subjekts, das Verfahren umfassend:
Kontaktieren einer Zieloberfläche (132) des Subjekts und eines Rahmens (110, 208, 300, 400) eines Gehäuses einer Einsatzvorrichtung (102, 202), während die transkutane Vorrichtung innerhalb des Gehäuses eingesetzt wird und während das Gehäuse verriegelt ist;
Bewegen eines Kolbens (108, 206) des Gehäuses zu der Zieloberfläche hin in einer ersten Richtung (134) relativ zu dem Rahmen, während der Rahmen in Kontakt mit der Zieloberfläche ist, und während sich der Rahmen in einer zweiten Richtung (136) relativ zu dem Kolben bewegt, wobei die zweite Richtung der ersten Richtung gegenüberliegt, wobei das Bewegen des Kolbens zu der Zieloberfläche hin in der ersten Richtung relativ zu dem Rahmen, während der Rahmen in Kontakt mit der Zieloberfläche ist, die transkutane Vorrichtung mindestens teilweise veranlasst, sich in Kontakt mit der Zieloberfläche zu bewegen;
Bewegen des Kolbens weg von der Zieloberfläche, nachdem sich die transkutane Vorrichtung in Kontakt mit der Zieloberfläche bewegt hat und während eine Feder (142) der Einsatzvorrichtung den Rahmen in die erste Richtung relativ zu dem Kolben bewegt;
Bewegen des Rahmens in die zweite Richtung relativ zu dem Kolben, nachdem der Kolben von der Zieloberfläche wegbewegt wurde, wobei das Bewegen des Rahmens in die zweite Richtung relativ zu dem Kolben, nachdem der Kolben von der Zieloberfläche wegbewegt wurde, das Gehäuse mindestens teilweise veranlasst, entriegelt zu werden;
Öffnen des Gehäuses nachdem sich das Gehäuse entriegelt hat; und
Entfernen der Feder aus dem Gehäuse nach dem Öffnen des Gehäuses.

11. Verfahren nach Anspruch 10, wobei:
das Öffnen des Gehäuses das Drehen einer Kappe (116) des Gehäuses relativ zu dem Kolben einschließt;
das Kontaktieren der Zieloberfläche und des Rahmens das Kontaktieren der Zieloberfläche und des Rahmens einschließt, während ein Anschlag (156) der Einsatzvorrichtung die Drehung der Kappe relativ zu dem Kolben blockiert; und
das Bewegen des Rahmens in die zweite Richtung relativ zu dem Kolben, nachdem der Kolben von der Zieloberfläche wegbewegt wurde, mindestens teilweise das Gehäuse veranlasst, sich durch Lösen des Anschlags zu entriegeln
und/oder
wobei das Entfernen der Feder ein Kippen des Gehäuses einschließt, um der Feder zu ermöglichen, durch Schwerkraft aus dem Gehäuse zu fallen.

12. Verfahren nach einem der Ansprüche 10 bis 11, wobei:
das Kontaktieren der Zieloberfläche und des Rahmens das Kontaktieren der Zieloberfläche und des Rahmens einschließt, während eine Nadel (126) der Einsatzvorrichtung innerhalb des Gehäuses angeordnet ist; und
das Bewegen des Kolbens zu der Zieloberfläche hin in der ersten Richtung relativ zu dem Rahmen, während der Rahmen mit der Zieloberfläche mindestens teilweise in Kontakt ist, die Nadel veranlasst, sich zu der Zieloberfläche und der Haut hin zu bewegen und diese zu durchstechen.
insbesondere
wobei:
das Bewegen des Kolbens zu der Zieloberfläche hin in der ersten Richtung relativ zu dem Rahmen, während der Rahmen mit der Zieloberfläche in Kontakt ist, die Nadel mindestens teilweise veranlasst, aus der Haut zurückzuziehen, um sich von der Zieloberfläche wegzubewegen und um sich in eine Patrone innerhalb des Gehäuse hineinzubewegen; und
das Verfahren ferner das Entfernen der Nadel und der Patrone aus dem Gehäuse nach dem Öffnen des Gehäuses umfasst.

13. Verfahren zum Verbinden einer transkutanen Vorrichtung mit der Haut eines Subjekts, das Verfahren umfassend:
Kontaktieren einer Zieloberfläche (132) des Subjekts und eines Rahmens (110, 208, 300, 400) eines Gehäuses einer Einsatzvorrichtung, während die transkutane Vorrichtung innerhalb des Gehäuses angeordnet ist;
Bewegen eines Kolbens (108, 206) der Einsatzvorrichtung zu der Zieloberfläche hin in einer ersten Richtung (134) relativ zu dem Rahmen, während der Rahmen in Kontakt mit der Zieloberfläche ist und während sich der Rahmen in einer zweiten Richtung (136) relativ zu dem Kolben bewegt, wobei die zweite Richtung der ersten Richtung gegenüberliegt, wobei das Bewegen des Kolbens zu der Zieloberfläche hin in der ersten Richtung relativ zu dem Rahmen während der Rahmen in Kontakt mit der Zieloberfläche ist, die transkutane Vorrichtung mindestens teilweise veranlasst, sich in Kontakt mit der Zieloberfläche zu bewegen;
Bewegen des Kolbens weg von der Zieloberfläche, nachdem sich die transkutane Vorrichtung in Kontakt mit der Zieloberfläche bewegt hat und während eine Feder (142) der Einsatzvorrichtung den Rahmen in die erste Richtung relativ zu dem Kolben bewegt;
Bewegen des Rahmens in die zweite Richtung relativ zu dem Kolben, nachdem der Kolben von der Zieloberfläche wegbewegt wurde;
Drehen des Rahmens in eine dritte Richtung (233) relativ zu dem Kolben, nachdem der Rahmen in die zweite Richtung relativ zu dem Kolben bewegt wurde, wobei die dritte Richtung senkrecht zu der ersten und der zweiten Richtung verläuft;
Trennen des Rahmens von dem Kolben nach dem Drehen des Rahmens in die dritte Richtung relativ zu dem Kolben; und
Entfernen der Feder aus dem Gehäuse, nachdem der Rahmen von dem Kolben getrennt wurde.

14. Verfahren nach Anspruch 13, ferner umfassend das Bewegen des Rahmens in die erste Richtung relativ zu dem Kolben, während der Rahmen von dem Kolben getrennt wird,
und/oder
wobei das Entfernen der Feder das Erlauben der Feder einschließt, aus dem Gehäuse durch die Schwerkraft zu fallen.

15. Verfahren nach einem der Ansprüche 13 bis 14, wobei das Bewegen des Kolbens zu der Zieloberfläche hin in einer ersten Richtung relativ zu dem Rahmen, während der Rahmen in Kontakt mit der Zieloberfläche ist, das Bewegen des Kolbens zu der Zieloberfläche hin in einer ersten Richtung relativ zu dem Rahmen einschließt, während der Rahmen in Kontakt mit der Zieloberfläche ist und während sich ein Keil (212) des Kolbens entlang eines Pfads (218, 224) innerhalb eines Kanals (214, 216, 302, 304, 402, 404) bewegt, der durch den Rahmen definiert ist,
insbesondere
wobei:
der Kanal und der Pfad ein erster Kanal (214, 302, 402) beziehungsweise ein erster Pfad (218) sind;
das Bewegen des Kolbens weg von der Zieloberfläche, nachdem sich die transkutane Vorrichtung in Kontakt mit der Zieloberfläche bewegt hat, das Bewegen des Kolbens weg von der Zieloberfläche einschließt, nachdem die transkutane Vorrichtung in Kontakt mit der Zieloberfläche bewegt wurde und während sich der Keil entlang des ersten Pfads innerhalb des ersten Kanals bewegt;
das Drehen des Rahmens in die dritte Richtung relativ zu dem Kolben, nachdem der Rahmen in die zweite Richtung relativ zu dem Kolben bewegt wurde, das Drehen des Rahmens in die dritte Richtung relativ zu dem Kolben einschließt, nachdem der Rahmen in die zweite Richtung relativ zu dem Kolben bewegt wurde und während sich der Keil entlang eines zweiten Pfads (224) innerhalb eines zweiten Kanals (216, 304, 404) bewegt, der durch den Rahmen definiert ist; und
der zweite Pfad von dem ersten Pfad abzweigt,
insbesondere
ferner umfassend ein blindes Ausrichten des Keils mit einer Mündung (228) des zweiten Kanals, während der Rahmen in der zweiten Richtung relativ zu dem Kolben bewegt wird, nachdem der Kolben von der Zieloberfläche weg bewegt wurde.

## Revendications

1. Dispositif de déploiement (102, 202), comprenant :
un boîtier (106, 204) comportant :
un piston (108, 206),
un cadre (110, 208, 300, 400) relié de manière mobile au piston et définissant une fenêtre de déploiement (120), et
un capuchon (116) relié de manière amovible au piston ;
un ressort (142) confiné à l'intérieur du boîtier par le capuchon, dans lequel le piston et le cadre sont reliés élastiquement l'un à l'autre par l'intermédiaire du ressort, et dans lequel le ressort pousse le cadre à se déplacer dans une première direction (134) par rapport au piston ;
un support (138) relié de manière mobile au cadre, dans lequel le support est conçu pour mettre un dispositif transcutané en contact avec une surface cible (132) d'un sujet par l'intermédiaire de la fenêtre de déploiement, au moins partiellement en réponse au déplacement du cadre dans une deuxième direction (136) par rapport au piston, la deuxième direction étant opposée à la première direction ; et
une butée (156) qui, lorsqu'elle est mise en prise, est conçue pour bloquer la séparation du capuchon du piston, dans lequel la butée est conçue pour se désolidariser au moins partiellement en réponse au déplacement du cadre dans la deuxième direction par rapport au piston.

2. Dispositif de déploiement selon la revendication 1, dans lequel le cadre porte la butée,
et/ou
dans lequel :
l'un parmi le piston et le capuchon comporte une surface d'étanchéité annulaire (170) ; et
l'autre parmi le piston et le capuchon comporte une bride annulaire (168) conçue pour venir en prise élastiquement avec la surface d'étanchéité lorsque le capuchon est relié au piston,
et/ou
dans lequel le ressort peut être retiré du boîtier lorsque le capuchon est séparé du piston.

3. Dispositif de déploiement selon l'une quelconque des revendications 1-2, dans lequel :
le capuchon et le piston comportent des filetages complémentaires (114) par lesquels le capuchon et le piston sont reliés l'un à l'autre de manière amovible ; et
la butée, lorsqu'elle est mise en prise, est conçue pour bloquer la rotation du capuchon par rapport au piston
et/ou
dans lequel :
le capuchon comporte un support (164) et une saillie (166) s'étendant vers l'extérieur du support dans un plan perpendiculaire à la première direction et à la deuxième direction ; et
la butée, lorsqu'elle est mise en prise, est adjacente circonférentiellement par rapport à la saillie le long d'un cercle à l'intérieur du plan.

4. Dispositif de déploiement (202), comprenant :
un boîtier (204) comportant :
un piston (206), et
un cadre (208, 300, 400) relié de manière mobile au piston et définissant une fenêtre de déploiement (120),
un ressort (142) confiné à l'intérieur du boîtier par le cadre, dans lequel le piston et le cadre sont reliés élastiquement l'un à l'autre par l'intermédiaire du ressort, et dans lequel le ressort pousse le cadre à se déplacer dans une première direction (134) par rapport au piston ;
un support (138) relié de manière mobile au cadre, dans lequel le support est conçu pour mettre un dispositif transcutané en contact avec une surface cible (132) d'un sujet par l'intermédiaire de la fenêtre de déploiement, au moins partiellement en réponse au déplacement du cadre dans une deuxième direction (136) par rapport au piston, la deuxième direction étant opposée à la première direction ; et
une butée (232) qui, lorsqu'elle est mise en prise, est conçue pour bloquer la séparation du cadre du piston, dans lequel la butée est conçue pour se désolidariser au moins partiellement en réponse au déplacement du cadre dans la deuxième direction par rapport au piston et à sa rotation ultérieure dans une troisième direction (233) par rapport au piston, la troisième direction étant perpendiculaire à la première et à la deuxième direction,
en particulier
dans lequel le cadre est conçu pour se séparer du piston au moins partiellement en réponse au déplacement du cadre dans la première direction par rapport au piston après avoir tourné dans la troisième direction par rapport au piston.

5. Dispositif de déploiement selon la revendication 4, dans lequel :
le cadre définit un canal (214, 216, 3 02, 3 04, 402, 404) s'étendant le long d'une trajectoire (218, 224) parallèle à la première et à la deuxième direction ;
le piston comporte une clavette (212) s'étendant dans le canal dans un plan perpendiculaire à la trajectoire ;
le support est conçu pour mettre le dispositif transcutané en contact avec la surface cible par l'intermédiaire de la fenêtre de déploiement, au moins partiellement, en réponse au déplacement du cadre dans la deuxième direction par rapport au piston ;
le cadre définit une plage de mouvement dans la deuxième direction par rapport au piston ; et
le déplacement du cadre dans la deuxième direction par rapport au piston sur toute l'amplitude du mouvement entraîne au moins partiellement le déplacement de la clavette à l'intérieur du canal entre une première extrémité (220) de la trajectoire et une seconde extrémité opposée (222) de la trajectoire.

6. Dispositif de déploiement selon la revendication 5, dans lequel :
le canal et la trajectoire sont un premier canal (214, 302, 402) et une première trajectoire (218), respectivement ; et
le cadre définit un second canal (216, 3 04, 404) s'étendant le long d'une seconde trajectoire (224) qui bifurque de la première trajectoire au niveau d'un point de bifurcation (226) situé entre la première et la seconde extrémité de la première trajectoire.

7. Dispositif de déploiement selon la revendication 6, dans lequel la butée est conçue pour se désolidariser au moins partiellement en réponse au déplacement du cadre dans la deuxième direction par rapport au piston pendant que la clavette se déplace le long d'une partie de la première trajectoire commençant au niveau de la première extrémité de la première trajectoire, et au déplacement ultérieur du cadre dans la troisième direction par rapport au piston pendant que la clavette se déplace le long d'une partie de la seconde trajectoire commençant au niveau du point de bifurcation,
en particulier
dans lequel :
le premier canal est fermé au niveau de la première extrémité de la première trajectoire ; et
le seconde canal est un canal traversant.

8. Dispositif de déploiement selon l'une quelconque des revendications 6-7, dans lequel la seconde trajectoire comporte une étape (308),
et/ou
dans lequel :
le second canal comporte une embouchure (228) au niveau du point de bifurcation ;
l'embouchure est espacée de la première extrémité de la première trajectoire d'au moins 10 % d'une longueur de la première trajectoire ; et
l'embouchure est espacée de la seconde extrémité de la première trajectoire d'au moins 10 % de la longueur de la première trajectoire .

9. Dispositif de déploiement selon l'une quelconque des revendications 6-8, dans lequel :
le second canal comporte une embouchure au niveau du point de bifurcation ; et
le cadre comporte :
un premier bouton (406) au niveau d'un premier côté de l'embouchure dans une dimension parallèle à la première trajectoire, et
un second bouton (408) au niveau d'un second côté opposé de l'embouchure dans la dimension parallèle à la première trajectoire,
en particulier
dans lequel le premier et le second bouton sont conçus pour guider la clavette loin de l'embouchure pendant que la clavette se déplace de la première extrémité de la première trajectoire à la seconde extrémité de la première trajectoire, pendant que la clavette se déplace de la seconde extrémité de la première trajectoire à la première extrémité de la première trajectoire, ou les deux à la fois.

10. Procédé de connexion d'un dispositif transcutané à la peau d'un sujet, le procédé comprenant :
la mise en contact d'une surface cible (132) du sujet et d'un cadre (110, 208, 300, 400) d'un boîtier d'un dispositif de déploiement (102, 202) pendant que le dispositif transcutané est placé à l'intérieur du boîtier et pendant le verrouillage du boîtier ;
le déplacement d'un piston (108, 206) du boîtier vers la surface cible dans une première direction (134) par rapport au cadre pendant que le cadre est en contact avec la surface cible et pendant que le cadre se déplace dans une deuxième direction (136) par rapport au piston, la deuxième direction étant opposée à la première direction, dans lequel le déplacement du piston vers la surface cible dans la première direction par rapport au cadre pendant que le cadre est en contact avec la surface cible entraîne, au moins partiellement, la mise en contact du dispositif transcutané avec la surface cible ;
l'éloignement du piston de la surface cible après que le dispositif transcutané soit entré en contact avec la surface cible et pendant qu'un ressort (142) du dispositif de déploiement déplace le cadre dans la première direction par rapport au piston ;
le déplacement du cadre dans la deuxième direction par rapport au piston après avoir éloigné le piston de la surface cible, dans lequel le déplacement du cadre dans la deuxième direction par rapport au piston après l'éloignement du piston de la surface cible entraîne au moins partiellement le déverrouillage du boîtier ;
l'ouverture du boîtier après que le boîtier se soit déverrouillé ; et
le retrait du ressort du boîtier après l'ouverture du boîtier.

11. Procédé selon la revendication 10, dans lequel :
l'ouverture du boîtier comporte la rotation d'un capuchon (116) du boîtier par rapport au piston ;
la mise en contact de la surface cible et du cadre comporte la mise en contact de la surface cible et du cadre pendant qu'une butée (156) du dispositif de déploiement bloque la rotation du capuchon par rapport au piston ; et
le déplacement du cadre dans la deuxième direction par rapport au piston après l'éloignement du piston de la surface cible entraîne au moins partiellement le déverrouillage du boîtier par désolidarisation de la butée
et/ou
dans lequel le retrait du ressort comporte l'inclinaison du boîtier pour permettre au ressort de tomber du boîtier par gravité.

12. Procédé selon l'une quelconque des revendications 10-11, dans lequel :
la mise en contact de la surface cible et du cadre comporte la mise en contact de la surface cible et du cadre lors du placement d'une aiguille (126) du dispositif de déploiement à l'intérieur du boîtier ; et
le déplacement du piston vers la surface cible dans la première direction par rapport au cadre pendant que le cadre est en contact avec la surface cible entraîne au moins partiellement le déplacement de l'aiguille vers la surface cible et la peau et le transpercement de celle-ci,
en particulier
dans lequel :
le déplacement du piston vers la surface cible dans la première direction par rapport au cadre pendant que le cadre est en contact avec la surface cible entraîne au moins partiellement le retrait de l'aiguille de la peau, son éloignement de la surface cible et son déplacement dans une cartouche à l'intérieur du boîtier ; et
le procédé comprend en outre le retrait de l'aiguille et de la cartouche du boîtier après l'ouverture du boîtier.

13. Procédé de connexion d'un dispositif transcutané à la peau d'un sujet, le procédé comprenant :
la mise en contact d'une surface cible (132) du sujet et d'un cadre (110, 208, 300, 400) d'un boîtier d'un dispositif de déploiement pendant que le dispositif transcutané est placé à l'intérieur du boîtier ;
le déplacement d'un piston (108, 206) du dispositif de déploiement vers la surface cible dans une première direction (134) par rapport au cadre pendant que le cadre est en contact avec la surface cible et pendant que le cadre se déplace dans une deuxième direction (136) par rapport au piston, la deuxième direction étant opposée à la première direction, dans lequel le déplacement du piston vers la surface cible dans la première direction par rapport au cadre pendant que le cadre est en contact avec la surface cible entraîne, au moins partiellement, la mise en contact du dispositif transcutané avec la surface cible ;
l'éloignement du piston de la surface cible après que le dispositif transcutané soit entré en contact avec la surface cible et pendant qu'un ressort (142) du dispositif de déploiement déplace le cadre dans la première direction par rapport au piston ;
le déplacement du cadre dans la deuxième direction par rapport au piston après l'éloignement du piston de la surface cible ;
la rotation du cadre dans une troisième direction (233) par rapport au piston après le déplacement du cadre dans la deuxième direction par rapport au piston, la troisième direction étant perpendiculaire à la première et à la deuxième direction ;
la séparation du cadre du piston après avoir fait tourner le cadre dans la troisième direction par rapport au piston ; et
le retrait du ressort du boîtier après avoir séparé le cadre du piston.

14. Procédé selon la revendication 13, comprenant en outre le déplacement du cadre dans la première direction par rapport au piston lors de la séparation du cadre du piston,
et/ou
dans lequel le retrait du ressort comporte le fait de laisser le ressort tomber du boîtier par gravité.

15. Procédé selon l'une quelconque des revendications 13-14, dans lequel le déplacement du piston vers la surface cible dans une première direction par rapport au cadre pendant que le cadre est en contact avec la surface cible comporte le déplacement du piston vers la surface cible dans une première direction par rapport au cadre pendant que le cadre est en contact avec la surface cible et pendant qu'une clavette (212) du piston se déplace le long d'une trajectoire (218, 224) à l'intérieur d'un canal (214, 216, 3 02, 304, 402, 404) défini par le cadre,
en particulier
dans lequel :
le canal et la trajectoire sont un premier canal (214, 302, 402) et une première trajectoire (218), respectivement ;
l'éloignement du piston de la surface de la cible après que le dispositif transcutané soit entré en contact avec la surface cible comporte l'éloignement du piston de la surface cible après que le dispositif transcutané soit entré en contact avec la surface cible et pendant que la clavette se déplace le long de la première trajectoire à l'intérieur du premier canal ;
la rotation du cadre dans la troisième direction par rapport au piston après avoir déplacé le cadre dans la deuxième direction par rapport au piston comporte la rotation du cadre dans la troisième direction par rapport au piston après avoir déplacé le cadre dans la deuxième direction par rapport au piston et pendant que la clavette se déplace le long d'une seconde trajectoire (224) à l'intérieur d'un second canal (216, 304, 404) défini par le cadre ; et
la seconde trajectoire se ramifie à partir de la première trajectoire,
en particulier
comprenant en outre l'alignement aveugle de la clavette avec une embouchure (228) du second canal lors du déplacement du cadre dans la deuxième direction par rapport au piston après avoir éloigné le piston de la surface cible.
